# EUROPEAN PATENT SPECIFICATION

(11) **EP 2 147 093 B1**
(45) Date of publication and mention of the grant of the patent: **03.07.2013**
(21) Application number: 08751455.0
(22) Date of filing: 13.05.2008
(51) Int. Cl.: C12N 1/21, C12P 21/00

(54) **PROCESS FOR OBTAINING BIOACTIVE RECOMBINANT PROTEIN FROM INCLUSION BODIES**
VERFAHREN ZUR GEWINNUNG EINES BIOAKTIVEN REKOMBINANTEN PROTEINS AUS EINSCHLUSSKÖRPERCHEN
PROCÉDÉ D'OBTENTION D'UNE PROTÉINE BIOACTIVE DE RECOMBINAISON À PARTIR DE CORPS D'INCLUSION

(30) Priority: 14.05.2007 IN DE10412007
(43) Date of publication of application: 27.01.2010
(73) Proprietor: National Institute of Immunology, New Delhi 110067 (IN)
(72) Inventor: PANDA, Amulya Kumar, New Delhi 110 067 (IN); SINGH, Mohan Surinder, New Delhi 110 067 (IN); UPADHYAY, Kumar Arun, New Delhi 110 067 (IN)
(74) Representative: Huenges, Martin
(86) International application number: PCT/IN2008/000297
(87) International publication number: WO 2008/139494

(56) References cited:
- US-A1- 2004 235 089
- KHAN FAAIZAH ET AL: "Alcohol-induced versus anion-induced states of alpha-chymotrypsinogen A at low pH" BIOCHIMICA ET BIOPHYSICA ACTA, vol. 1481, no. 2, 29 September 2000 (2000-09-29), pages 229-236, XP004279004 ISSN: 0006-3002
- CLARK E D B: "Protein refolding for industrial processes" CURRENT OPINION IN BIOTECHNOLOGY 20010401 GB, vol. 12, no. 2, 1 April 2001 (2001-04-01), pages 202-207, XP002496443 ISSN: 0958-1669
- CABRITA L D ET AL: "PROTEIN EXPRESSION AND REFOLDING - A PRACTICAL GUIDE TO GETTING THE MOST OUT OF INCLUSION BODIES" BIOTECHNOLOGY ANNUAL REVIEW, ELSEVIER, vol. 10, no. SPEC. ISSUE, 1 January 2004 (2004-01-01), pages 31-50, XP009045245 ISSN: 1387-2656
- VALLEJO L F ET AL: "Strategies for the recovery of active proteins through refolding of bacterial inclusion body proteins" MICROBIAL CELL FACTORIES 20040902 GB, vol. 3, 2 September 2004 (2004-09-02), XP002496444 ISSN: 1475-2859
- SINGH ET AL: "Solubilization and refolding of bacterial inclusion body proteins" JOURNAL OF BIOSCIENCE AND BIOENGINEERING, ELSEVIER, AMSTERDAM, NL, vol. 99, no. 4, 1 April 2005 (2005-04-01), pages 303-310, XP005665508 ISSN: 1389-1723
- PANDA AMULYA K: "BIOPROCESSING OF THERAPEUTIC PROTEINS FROM THE INCLUSION BODIES OF ESCHERICHIA COLI" ADVANCES IN BIOCHEMICAL ENGINEERING, BIOTECHNOLOGY, SPRINGER, BERLIN, DE, vol. 85, 1 January 2003 (2003-01-01), pages 43-93, XP009083326 ISSN: 0724-6145

## Description

### FIELD OF INVENTION

The present investigation relates to a process for obtaining bioactive recombinant protein from inclusion bodies.

### BACKGROUND OF THE INVENTION

Expression of recombinant protein as inclusion bodies provides multiple advantages such as easy separation of large quantity of highly enriched protein, less protein degradation by cell proteases and reduced toxicity to the host cells.(M.M. Carrio, A. Villaverde, Protein aggregation as bacterial inclusion bodies is reversible, FEBS Lett. 489, 2001, 29-33; S. Ventura, A. Villaverde, Protein quality in bacterial inclusion bodies, Trends Biotechnol. 24, 2006, 179-185). Most notable characteristics of inclusion body protein aggregates are the existence of native-like secondary structure of the expressed protein (K. Oberg, B.A. Chrunyk, R. Wetzel, A.L. Fink, Nativelike secondary structure in interleukin-1 beta inclusion bodies by attenuated total reflectance FTIR, Biochemistry 33, 1994, 2628-2634, T.M. Przybycein, J.P. Dunn, P. Valax, G. Georgiou, Secondary structure characterization of beta-lactamase inclusion bodies, Protein Eng 7,1994, 131-136; D. Ami, A. Natalello, G. Taylor, G. Tonon, D.S. Maria, Structural analysis of protein inclusion bodies by Fourier transform infrared microspectroscopy, Biochim.Biophys.Acta 1764, 2006, 793-799) and the specificity of intermolecular interaction among a single type of protein molecule to form aggregates (A.M. Speed, D.I. Wang, J. King, Specific aggregation of partially folded polypeptide chains: the molecular basis of inclusion body composition, Nat. Biotechnol. 14, 1996, 1283-1287; R.S. Rajan, M.E. Illing, N.F. Bence, R.R. Kopito, Specificity in intracellular protein aggregation and inclusion body formation, Proc. Natl. Acad. Sci. U.S.A 98, 2001, 13060-13065).

Expression of a recombinant protein in *E. coli* leads to accumulation of partially folded protein intermediates into non bioactive intracellular refractile inclusion body proteins.

Solubilization of inclusion body proteins using mild condition without disturbing the existing native-like secondary structure is thus more important for improved recovery of bioactive proteins (R.H. Khan, K.B. Rao, A.N. Eshwari, S.M. Totey, A.K. Panda, Solubilization of Recombinant Ovine growth hormone with retention of native-like secondary structure and its refolding from the inclusion bodies of Escherichia Coli, Biotechnol. Prog. 14, 1998, 722-728; S.M. Singh, A.K. Panda, Solubilization and refolding of bacterial inclusion body proteins, J.Biosci.Bioeng. 99; 2005; 303-310). High pressure (1-2 k bar) has synergistically been used in combination with non denaturing concentration of guanidine hydrochloride for solubilization and refolding of hGH, β-lactamase and lysozyme (R.J. St. John, J.F. Carpenter, T.W. Randolph, High pressure fosters protein refolding from aggregates at high concentrations, Proc.Natl.Acad.Sci.U.S.A 96, 1999, 13029-13033). Low concentration of guanidine hydrochloride and L-arginine has also been used for solubilization and refolding of inclusion bodies of green fluorescent protein (K. Tsumoto, M. Umetsu, I. Kumagai, D. Ejima, T. Arakawa, Solubilization of active green fluorescent protein from insoluble particles by guanidine and arginine, Biochem.Biophys.Res.Commun. 312, 2003, 1383-1386). High pH (pH 12.5) along with 2 M urea has also been used for solubilization and refolding of human growth hormone inclusion bodies (A.K. Patra, R. Mukhopadhyay,R. Mukhija, A. Krishnan, L.C. Garg, A.K. Panda, Optimization of inclusion body solubilization and renaturation of recombinant human growth hormone from Escherichia coli, Protein Expr.Purif. 18, 2000, 182-192).

In spite of many successful refolding procedures of inclusion body proteins, the renaturation process for each protein is different, most of the time carried out in empirical way and contributes significantly to the cost of production. One of the major problems associated with low recovery of bioactive protein from inclusion bodies is protein aggregation during renaturation process.

Renaturation of inclusion body proteins into bioactive form is cumbersome, results in low recovery of the final product and also accounts for the major cost in overall production of recombinant proteins using *E. coli* as a host (A.K. Panda, Bioprocessing of therapeutic proteins from the inclusion bodies of Escherichia coli, Adv.Biochem.Eng/Biotechnol, 85, 2003, 43-93; G. Walsh, Biopharmaceutical benchmarks 2006, Nat .Biotechnol. 24, 2006, 411-421; F.A. Marston, The purification of eukaryotic polypeptides synthesized in Escherichia coli, 240, 1986, 1-12; B. Fahnert, H. Lilie, P. Neubauer, Inclusion bodies: formation and utilization, Adv.Biochem.Eng Biotechnol. 89, 2004, 93-142). In general, proteins expressed as inclusion bodies in *E. coli* are solubilized by the use of high concentration of chaotropic solvents and the soluble proteins are then refolded to their native state after removing the chaotropic agents or other salts by dialyzing the proteins in buffers containing reducing and oxidizing agents (E.D. Clark, Protein refolding for industrial processes, Curr. Opin. Biotechnol. 12, 2001, 202-207; A. P. Middleberg, Preparative protein folding, Trends in Biotechnology, 20, 2002, 437-443; R.V. Datar, T. Cartwright, C.G. Rosen, Process economics of animal cell and bacterial fermentations: a case study analysis of tissue plasminogen activator, Biotechnology (N.Y.) 11, 1993, 349-357; H. Lilie, E. Schwarz, R. Rudolph, Advances in refolding of proteins produced in E. coli, Curr. Opin.Biotechnol. 9, 1998, 497-501). Solubilization by chaotropic salt disturbs proteins secondary structure (C.E. De Bemardez, E. Schwarz, R. Rudolph, Inhibition of aggregation side reactions during in vitro protein folding, Methods Enzymol. 309, 1999, 217-236; K.A. Dill, D. Shortle, Denatured states of proteins, Annu.Rev.Biochem. 60, 1991, 795-825) and exposes the hydrophobic patches of the polypeptide chain which have more propensities to aggregate with each other. Because of these two above factors, most of the times the overall yield of bioactive proteins from inclusion bodies are around 15-25 % of the total expressed protein. Thus there is need to develop an efficient method for obtaining higher amount of bioactive protein from inclusion bodies.

### SUMMARY OF THE INVENTION

The present investigation relates to a process for obtaining bioactive recombinant protein from inclusion bodies. The process comprises solubilization of inclusion body protein using 2M urea and 4M to 8M propanol.

One aspect of the present invention relates to a process for obtaining bioactive recombinant protein from inclusion bodies, the process comprising solubilizing inclusion bodies in a solubilization buffer comprising Tris, urea and propanol to obtain a suspension; incubating the suspension at room temperature; and adding refolding buffer comprising Tris, urea and sucrose to the suspension to obtain bioactive recombinant protein, wherein said solubilization buffer comprises 2M urea and 4M to 8M propanol.

### BRIEF DESCRIPTION OF THE ACCOMPANYING DRAWING

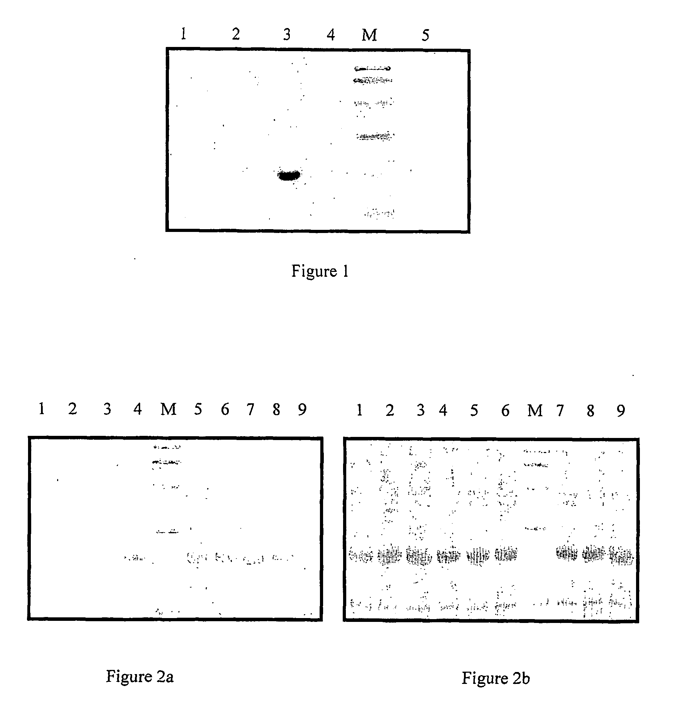

**Figure 1** shows SDS-PAGE analysis ofr-hGH IBs solubilized in 6 M concentration of different alkyl alcohols along with 2 M urea. Lane 1-5: Inclusion Bodies solubilized in methanol, ethanol, n-propanol, ethylene glycol, glycerol respectively. Lane M is LMW marker (97, 66, 45, 30, 20.1, 14.4 kDa).
**Figure 2:** a) SDS-PAGE analysis of r-hGH Inclusion Bodies (IBs) solubilized in increasing concentration (1-8 M) of 6 M n-propanol along with 2 M urea; Lane 1-9: IBs solubilized in 1-8 M n-propanol and buffer without n-propanol along with 2 M urea respectively; b) SDS-PAGE analysis of pellets after solubilization of IBs in increasing concentration (1-8 M) of n-propanol along with 2 M urea; Lane 1-9: pellets after solubilization in increasing concentration (1-8 M) of n-propanol and buffer without n-propanol respectively. Lane M is LMW marker (97, 66, 45, 30, 20.1, 14.4 kDa).

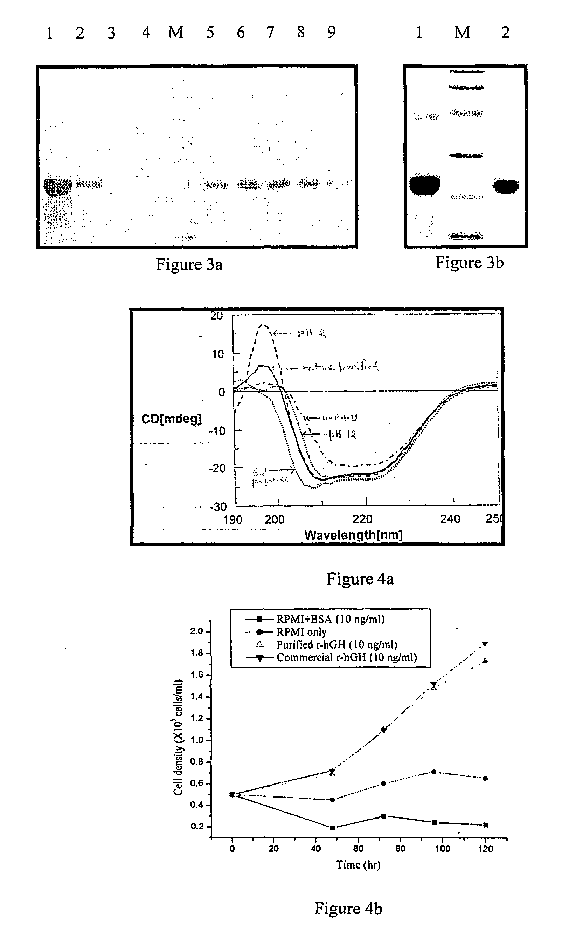

**Figure 3:** a) SDS-PAGE analysis of r-hGH purified after DEAE-anion exchange chromatography; Lane 1-4: solubilized, refolded, flow through and wash respectively;Lane 5-9: elutes after anion exchange chromatography; b) SDS-PAGE analysis of r-hGH purified after S-100 size exclusion chromatography. Lane 1 is load and lane 2 is pooled elutes; Lane M is LMW marker (97, 66, 45, 30, 20.1, 14.4 kDa)
**Figure 4:** a) Far UV CD spectra of purified r-hGH solubilized in different buffers pH 2 , 12 are r-hGH solubilied in pH 2 and pH 12 buffers, 6 M n-propanol: r-hGH solubilized in 6 M n-propanol, n-P+U is r-hGH solubilized in 6 M n-propanol with 2 M urea, native purified is commercially available hGH; 4b) Growth promoting activity of purified r-hGH in comparison to commercially available r-hGH

### DESCRIPTION OF THE INVENTION

The present invention relates to a process for obtaining bioactive recombinant protein from inclusion bodies without unfolding it completely into random coil structure. The process comprises solubilization of inclusion bodies using 2M urea and 4M to 8M propanol.

The present invention provides a process of solubilization of inclusion body aggregates without disturbing the existing native like secondary structure of the protein. The process disclosed in the present invention comprises solubilization of inclusion bodies (protein aggregates) in a mixture of 2M n-propanol an 4M to 8M urea. The process of solubilizing inclusion bodies disclosed in the present invention is very mild as compared with the methods known in the art that comprises solubilization of inclusion bodies in a buffer having high concentration of either chaotropic agents like Urea (8M) and GdmCl (6M) or detergents.

The process of solubilization disclosed in the present invention is not only safe but helps in improved recovery of bioactive protein from inclusion body proteins. The solubilization buffer disclosed in the present invention protects the secondary structure of the protein during solubilization and help in reducing aggregation during refolding of the protein.

The problem solved by the present invention is to obtain high yield of bioactive recombinant protein from inclusion bodies. The process of solubilization of inclusion body proteins disclosed in the present invention is easy and results in high throughput recovery of bioactive protein from inclusion bodies.

The process of solubilization of inclusion body proteins disclosed in the present invention can also be applicable to solubilize the inclusion body proteins expressed in varieties of expression system such as *E. coli,* yeast and eukaryotic cells such as an insect cell(s), animal cell(s) and plant cell(s).

In the present invention, a recombinant protein expressed as inclusion bodies in E. *coli* was isolated and the bioactive protein was obtained using the process disclosed in the present invention. Inclusion body protein of various recombinant proteins such as human growth hormone, bovine growth hormone and human *superoxide dismutase* can be solubilized using the process disclosed in the present invention.

Recombinant proteins expressed as inclusion bodies in *E. coli* were isolated and solubilized in 6M n-Propanol and 2M Urea in 50mM Tris buffer at pH 8.5. The solubilized proteins were refolded using pulsatile dilution process where the protein was diluted 10 fold in Tris buffer at pH 8.5 containing 2M urea. This refolded sample was subjected to ion exchange chromatography without any dialysis. Recombinant protein thus obtained was further purified using DEAE sepharose anion exchange chromatography. The purified proteins were similar in physicochemical and biological properties. Solubilization of protein aggregates using suitable mild condition which does not unfold the protein completely during solubilization helped in better recovery of bioactive protein. Various recombinant proteins such as human growth hormone, bovine growth hormone, human *super oxide dismutase* (SOD) and *Mycobacterium tuberculosis* derived recombinant *enolase* and *pyruvate kinase* can be obtained in bioactive form using this process.

### Solubilization of inclusion bodies of recombinant human growth hormone

Inclusion bodies of recombinant human growth hormone (r-hGH) were solubilized with different alkyl alcohol. It was observed that human growth hormone inclusion bodies could be solubilized in 6 M propanol. The solubilization was lower than achieved with 8 M urea. It was observed that use of very little urea (2 M) improved the solubilization of human growth hormone inclusion bodies. As the n-propanol concentration was increase from 1 M to six molar the percent solubility of human growth hormone increases from 5% to 97 %. Maximum solubilization of inclusion bodies was achieved when 6 M propanol was used in combination of 2 M urea. More 98 % of the inclusion body aggregates could be solubilized from about 4 mg/ml protein concentration of inclusion body protein.

### Solubilization of different inclusion body proteins

Different inclusion body proteins such as human growth hormone, bovine growth hormone, human *super oxide dismutase* (SOD) and *Mycobacterium tuberculosis* derived recombinant *enolase* and *pyruvate kinase* were solubilized in buffer containing 6M propanol in combination of 2 M urea. It was observed that almost all the recombinant protein of diverse origin was solubilized in the buffer. Solubilization of all the recombinant proteins was comparable to that achieved with 8 M urea solution.

### Solubilization and refolding of Human Superoxide dismutase

Human superoxide dismutase (SOD) was expressed as inclusion bodies in *E.coli.* The recombinant SOD was solubilized and refolded in to bioactive form using this method. Fed-batch fermentation of *E. coli* resulted in production of 2 g/l of recombinant SOD as inclusion bodies. The denatured recombinant SOD in form of inclusion bodies were isolated and purified from the cell by the lysis and centrifugation. The inclusion body pellet was re-suspended in 6 M n-propanol in presence of 2 M urea solution. The solubilized recombinant SOD was separated by high speed centrifuge. Maximum solubility of recombinant SOD in propanol based buffer was 3 mg/ml. The protein was then refolded and purified. The propanol based solubilization of inclusion body protein results in high recovery of bioactive SOD.

Similar solubilization protocol using n-propanol with 2 M urea has been successfully used for solubilization of proteins aggregates formed during high level expression of recombinant proteins. Examples of proteins solubilized are recombinant ovine growth hormone, asparaginase, enolase, aldolase and pyruvate kinase expressed as inclusion bodies in E. *coli.* The solubilized aggregate could be refolded using standard refolding procedures. As the above solubilization process is based on disruption of hydrophobic interaction and disulfide bond formation. Such solubilization process can thus be used to solubilized proteins aggregates formed during high level expression of recombinant proteins such as growth factors, interferons, interleukins, hormones and antibody fragments.

### Existence of native like secondary structure during solubilization

Solubilized r-hGH in propanol was checked for the presence of secondary structure CD spectroscopy analysis. It was observed that human growth hormone in presence of 6 m propane or propanol with 2 M urea retains native-like secondary structure. The peak around 210 and 222 showed that the proteins have retained their secondary structure during solubilization. Human growth hormone inclusion bodies solubilized at low pH also show the presence of native like secondary structure. This is in accordance with the existence of native like secondary structure of protein as reported for β lactamase and as interleukins. Thus inclusion body aggregates was solubilized in propanol while retaining the existing secondary structure of the protein.

### Solubilization and refolding of human growth hormone in propanol

Different concentration of inclusion body proteins were used to check the maximum solubility of human growth hormone propanol. It was observed that as high as 4 mg/ml of inclusion body protein could be solubilized in the buffer. This was lower than the solubilization capacity of strong denaturants like urea or guanidium hydrochloride. However as described previously, this protected the native like secondary structure during solubilization. This was used for subsequent solubilization and refolding.

The solubilized protein were very easily refolded resulting in little aggregation. Ten fold dilution of the organic solvent did not promote aggregations observed with urea or guanidine hydrochloride based protein solubilization. Solubilized protein was loaded on ion exchange column and the protein was purified using standards elution procedures used for recombinant human growth hormone. The recovery of human growth hormone from such solubilization step was about 50 %. Thus protein solubilized using high concentration of propanol was successfully refolded with very little aggregation and was subsequently purified.

### Purification of recombinant hGH

The solubilized r-hGH was diluted 10X and pH of the buffer was adjusted to 8.5. Purification of solubilized r-hGH refolded through rapid dilution resulted 13 mg of the refolded protein after ion exchange and gel filtration chromatography. Aggregation during pulsatile renaturation process was found to be negligible in comparison to buffer exchange method by gel filtration or rapid buffer exchange method. Solubilized and refolded r-hGH from pulsatile renaturation process was passed through DEAE-Sepharose column for purification. Chromatogram from DEAE sepharose column showed the presence of two peaks, the first one predominantly constituting pure r-hGH (Figure 4a, b). The r-hGH eluted in first peak first peak homogeneous and was around 70 % of the total protein. However, in the second peak some amount of r-hGH was co-eluted along with r-hGH dimer which constituted about 25-30% of the total protein. Dimers eluted from the ion exchange column were further separated using S-100 gel filtration column which further improved the overall recovery of the r-hGH. Chromatogram of S-100 column showed the presence of pure r-hGH as a single homogenous protein. The overall yield of the purified refolded r-hGH from the inclusion bodies of E. *coli* was about 50 % using pulsatile dilution method, 26 % and 40 % using rapid dilution and gel filtration chromatography protocol respectively.

### Characterization of purified protein

The purified refolded r-hGH was characterized using CD, Fluorescence, RIA, RRA and western blotting. Even though these methods gave different recovery of the solubilized protein, the r-hGH refolded by all these methods was same. The r-hGH without having histidine tag behaved similar with its histidine counterpart on both physico/chemical and biological properties. CD spectra showed the characteristics pattern of alpha helix structure and the fluorescence spectrum showed a peak at 332 nm representative of hGH tertiary structure. Radio receptor assay of the refolded r-hGH was similar to that of the native hGH thus authenticating the bioactive conformation of the purified r-hGH.

Inclusion bodies of r-hGH were isolated to more than 90% purity by extensive washing in 2% deoxycholate in 50 mM Tris buffer pH 8.5.

In order to protect the native-like secondary structure, r-hGH inclusion bodies were solubilized in buffer conating 6 M propanol and 2M urea. Use of propanol along with 2M urea significantly improved r-hGH solubilization from inclusion bodies without disturbing the existing native-like secondary structure of the proteins. The overall yield of the r-hGH from the inclusion bodies was ∼50 % in comparison to 20 to 25 % yield when the inclusion bodies were solubilized in high concentration of chaotropic reagents.

### Solubilization of different inclusion body proteins

Different inclusion body proteins were solubilized in buffer containing 6M propanol in combination of 2 M urea. It was observed that almost all the recombinant protein of diverse origin were soluble in n-propanol. Solubilization of all the recombinant proteins was comparable to that achieved with 8 M urea solution. Solubilization of inclusion body aggregates of Human SOD, bovine growth hormone, *Mycobacterium tuberculosis* derived recombinant enolase, pyruvate kinase could be solubilized in the organic solvent base buffers.

In accordance with the present invention, a process of solubilization of inclusion bodies is provided, wherein the inclusion bodies were solubilized in a solubilization buffer comprising Tris 2M urea, 4M to 8M propanol and sucrose.

One embodiment of the present invention provides a process for obtaining bioactive recombinant protein from inclusion bodies, the process comprising solubilizing inclusion bodies in a solubilization buffer comprising Tris, 2M urea and 4M to 8M propanol to obtain a suspension; incubating the suspension at room temperature: and adding refolding buffer comprising Tris, urea and sucrose to the suspension to obtain bioactive recombinant protein

Another embodiment of the present invention provides the process for obtaining bioactive recombinant protein from bodies, wherein the recombinant protein is expressed in a host cell selected from a group consisting of *E. coli,* yeast and eukaryotic cell.

Yet another embodiment of the present invention provides the process for obtaining bioactive recombinant protein from inclusion bodies, wherein the recombinant protein can be expressed in a eukaryotic cell selected from a group consisting of an insect cell, an animal cell and a plant cell.

Still yet another embodiment of the present invention provides the process for obtaining bioactive recombinant protein from inclusion bodies, wherein the recombinant protein is selected from a group consisting of growth factors, interferons, interleukins, hormones and antibody fragments.

Still yet another embodiment of the present invention provides the process for obtaining bioactive recombinant protein from inclusion bodies, wherein the recombinant protein is selected from a group consisting of human growth hormone, bovine growth hormone; human *super oxide dismutase* (SOD) and *Mycobacterium tuberculosis* derived recombinant *enolase* and *pyruvate kinase.*

Another embodiment of the present invention provides the process for obtaining bioactive recombinant protein from inclusion bodies, wherein the inclusion body protein(s) was solubilized in a solubilization buffer comprising 50-100 mM Tris, 2 M urea, 6 M n-Propanol, 5-10 % sucrose, 0.5 mM EDTA and 1mM PMSF.

In one embodiment, the present invention provides the solubilization buffer comprising Trisl, 2M urea and 4M to 8M n-Popanol, wherein pH of the buffer is about 8.5.

In one embodiment, the present invention provides the solubilization buffer comprising Tris, 2M urea, 4M to 8M n-Propanol and sucrose, wherein pH of the buffer is about 8.5, wherein the buffer is used for solubilization of inclusion bodies.

In another embodiment, the present invention provides the solubilization buffer comprising 50-100 mM Tris, 2 M urea, 6 M n-Propanol, 5-10 % sucrose, 0.5 mM EDTA and 1mM PMSF, wherein pH of said buffer is about 8.5.

In another embodiment, the present invention provides the solubilization buffer comprising 50-100 mM Tris, 2 M urea and 6 M n-Propanol.

In another embodiment, the present invention provides the solubilization buffer comprising 50-100 mM Tris, 2 M urea, 6 M n-Propanol, 5-10 % sucrose, 0.5 mM EDTA and 1mM PMSF, wherein pH of said buffer is about 8.5, wherein the buffer is used for solubilization of inclusion bodies.

In another embodiment, the present invention provides the process for obtaining bioactive recombinant protein from inclusion bodies, wherein the inclusion bodies were solubilized in a solubilization buffer comprising Tris, urea and propanol to obtain a suspension and incubated the suspension at room temperature for about 30 minutes.

The process for obtaining bioactive recombinant protein from inclusion bodies, wherein refolding of the solubilized protein is carried out in the refolding buffer comprising Tris, urea and sucrose.

The process for obtaining bioactive recombinant protein from inclusion bodies, wherein refolding of the solubilized protein is carried out in the refolding buffer comprising Tris, urea and sucrose, wherein pH of the refolding buffer is about 8.5.

The process for obtaining bioactive recombinant protein from inclusion bodies, wherein refolding of the solubilized protein is carried out in the refolding buffer comprising 50-100 mM Tris, 5 mM EDTA, 2M urea, 5-10% sucrose and 1mM PMSF.

The process for obtaining bioactive recombinant protein from inclusion bodies, wherein refolding of the solubilized protein is carried out in the refolding buffer comprising 50-100 mM Tris, 5 mM EDTA, 2M urea, 5-10% sucrose and 1mM PMSF, wherein pH of the refolding buffer is about 8.5.

Surprisingly more than 40 % bioactive recombinant protein was recovered using the process of solubilization of inclusion bodies and refolding of the solubilized proteins provided by the present invention.

The process for obtaining bioactive recombinant protein from inclusion bodies disclosed in the present invention is very efficient, wherein the overall yield of the bioactive recombinant protein from inclusion bodies is about 50% as compared to the conventional process that results in 20 to 25% overall yield of recombinant protein.

The process of solubilization and purification of bioactive proteins from inclusion bodies could be used for maximizing recovery of therapeutically important proteins from as inclusion bodies expressed in various expression system such as E. *coli.*

### EXAMPLES

The following examples are put forth so as to provide those of ordinary skill in the art with a complete invention and the description of how to make and use the present invention.

### Example 1

### Expression of r-hGH

The fragment of r-hGH without any histidine tag was cloned in pQE 60 vector, which had phenylalanine in the second codon position following the start site ATG (A.K. Patra, R. Mukhopadhyay, R. Mukhija, A. Krishnan, L.C. Garg, A.K. Panda, Optimization of inclusion body solubilization and renaturation of recombinant human growth hormone from Escherichia coli, Protein Expr. Purif. 18, 2000, 182-192). E. *coli* M15 cells containing the recombinant expression plasmid pQE 60- r-hGH were grown in LB medium containing 25 µg/ml kanamycin and 100 µg/ml ampicillin. The culture was induced with 1 mM IPTG and was grown further for 4 hours. Expression of r-hGH in the total cell extracts from both un-induced and induced cultures was checked by SDS-PAGE.

### Example 2

### Isolation and purification of r-hGH inclusion bodies

Induced recombinant *E. coli* cells expressing r-hGH harvested from fed-batch fermentation process were centrifuged at 6,000 rpm for 30 minutes and the pellet was dissolved in 50 mM Tris-HCl buffer (pH 8.5) containing 5 mM EDTA and 1 mM PMSF. E. *coli* cells (5 gram wet weight cells containing approximately 50-60 mg r-hGH in the form of inclusion bodies) were lysed by sonication and centrifuged at 12,000 rpm for 20 min to isolate r-hGH inclusion bodies. The pellet obtained was washed twice with 50 mM Tris-HCl buffer (pH 8.0) containing 5 mM EDTA and 2% deoxycholate and kept at 37°C, overnight for solubilization of membrane proteins by the detergent (R.H. Khan, K.B. Rao, A.N. Eshwari, S.M. Totey, A.K. Panda, Solubilization of Recombinant Ovine growth hormone with retention of native-like secondary structure and its refolding from the inclusion bodies of Escherichia Coli. Biotechnol. Prog. 14 (1998) 722-728). The inclusion bodies were finally washed with distilled water to remove any contaminating salts and detergent, centrifuged at 12,000 rpm for. 20 min and the pellet was used for estimation of r-hGH. At this stage, inclusion bodies contained mostly r-hGH (>90%), the majority in the form of monomer around 22 kDa in SDS-PAGE. The inclusion bodies were completely soluble in 100 mM Tris-HCl buffer (pH 8.5) containing 1% SDS and the solubilized r-hGH samples were diluted appropriately and estimated using BCA protein assay. SDS-PAGE was carried out using method described by Laemmli (U.K. Laemmli, Cleavage of structural proteins during the assembly of the head of bacteriophage T4, Nature 227, 1970, 680-685).

### Example 3

### Solubilization of r-hGH inclusion bodies

### Organic solvents screening

Inclusion bodies of hGH were solubilized in six different solubilization buffers. Compositions of the solubilization buffer are provided in Table 1. Homogenous mixture of the inclusion bodies (30 µl) suspension was mixed with 90 µl of 1.33 X solubilization buffer. Suspension was incubated at room temperature for 30 minutes and centrifuged at 13000 rpm for 10 minutes at 4°C. Supernatant was collected and 5 µl of this supernatant was subjected to SDS-PAGE analysis (Figure 1) and 50 µl was subjected to TCA precipitation. TCA precipitate was solubilized in 1ml solubilization buffer of pH 12.5 comprising 50 mM Tris, 8 M urea and OD of this solution was measured at 280 nM.

### Optimization of n-Propanol Concentration

Homogenous IBs suspension (30 µl) was mixed with 90 µl of 1.33X solubilization buffer with increasing concentration of n-Propanol (1-8 M). Suspension was incubated at room temperature for 30 minutes and was centrifuged at 13000 rpm for 10 minutes at 4°C. Supernatant was collected and 5 µl of this supernatant was subjected to SDS-PAGE analysis (Figure 2 a, 2 b).

Purified r-hGH inclusion bodies were solubilized in 100 mM Tris buffer at pH 8 with high concentration of ethanol, methanol, propanol and butanol with 2M urea. The suspension was vortexed and left for 30 min at room temperature and the turbidity of the solution was measured at 450 nm. The samples were again centrifuged at 12000 rpm for 10 min. The supernatant after filtering through a 0.45 µm Millipore filter was measured at 280 nm for an estimation of protein content. The supernatant was analyzed on SDS PAGE and was subsequently used for refolding and purification.

### Comparative refolding vield

Inclusion bodies of hGH were prepared from 2 litre bacterial culture by normal DOC washing method. Final pellets of inclusion bodies were re-suspended in 1 ml MQ water. Of these IBs suspension 10 µl was used for micro BCA protein estimation. IBs suspension (150 µl) was solubilized in 450 µl of different (1.33X) solubilization buffers. Solubilized samples were incubated at room temperature for 30 minutes. After incubation, solubilized IBs were centrifuged at 13000 rpm for 10 minutes at 10°C. Soup was collected in a fresh eppendorf. 1.25 µl of this soup was loaded over SDS-PAGE. Of this 50 µl of soup was taken for TCA precipitation. TCA precipitate was solubilized in 1 ml. solubilization buffer (Tris 50 mM, pH 12.5, urea 8 M). OD at 280 nm of this solubilized sample was measured. This OD at 280 nm was used to normalize the amount of protein taken for refolding studies. Calculation was made so that equal amount of protein was taken for refolding in different refolding buffers. Refolding was done at ice by pulsatile dilution in an installment of 10 µl per minute. One ml of refolded soup was subjected to OD measurement at 480 nm whereas rest of refolded soup was centrifuged at 13000 rpm for 10 minutes at 4°C. 500 µl of centrifuged soup was subjected to TCA precipitation. TCA precipitate was solubilized in 1 ml. solubilization buffer (Tris 50 mM, pH 12.5, urea 8 M). Of this centrifuged refolded soup 10 µl was used for SDS-PAGE analysis. Comparative refolding of r-hGH after solubilization in various solubilization buffers is provided in Table 2.

### Example 4

### Solubilization and refolding of Inclusion bodies

Inclusion bodies (2.5 ml) was solubilized in 22.5 ml solubilization buffer (50 mM Tris, 6 M n-Propanol, 2 M Urea, 10% sucrose, 0.5 mM EDTA, 1mM PMSF, pH 8.5) to a final volume of 25 ml. Solubilization mixture was incubated at room temperature for 30 minutes and was centrifuged at 16000 rpm for 30 minutes. Supernatant was collected and 100 µl of solubilized protein soup was dialyzed against 2 % SDS. Protein concentration of this dialyzate was measured by micro BCA protein estimation kit (Pierce). Remaining solubilized soup was subjected to pulsatile refolding. 25 ml of solubilized soup was diluted to a final volume of 250 ml at an installment rate of 0.1 ml/minute (50mM Tris, 2M Urea, 10% Sucrose, 0.5 mM EDTA, 1mM PMSF pH 8.5, 1mM oxidized glutathione, 5 mM reduced glutathione). Refolded sample was centrifuged at 12 k rpm at 4°C for 30 minutes. Soup was collected and stored at 4°C and 100 µl of refolded protein soup was dialysed against 2 % SDS. Protein concentration of this dialyzate was measured by micro BCA protein estimation kit (Pierce).

### Example 5

### Purification of hGH by column chromatography

### Axial column chromatography

50 ml of DEAE fast flow media was packed in XK-16/40 with a flow rate of 2 ml/min. Column was first washed with 250 ml MQ at a flow rate of 2ml/min. Column was equilibrated with 250 ml of equilibration buffer (50 mMTris, 2 M Urea, 10% Sucrose, 0.5mM EDTA, 1mM PMSF pH 8.5) at a flow rate of 2 ml/min. Refolded r-hGH (250 ml) was loaded onto the column at a flow rate of 2ml/min. Column was washed with 250 ml of equilibration buffer at a flow rate of 2ml/min. Bound protein was eluted using a NaCl gradient (0 M to 500 mM) at a flow rate of 2 ml/min. Column was subsequently deproteinated and regenerated with 200 ml 2 M NaCl and 200 ml 0.5 M NaOH respectively. Homogeneity of eluted protein was checked by SDS-PAGE and yield was calculated by micro BCA (Pierce) (Figure 3 and Figure 4).

### Gel filtration chromatography

Pooled elutes form Ion Exchange chromatography were dialyzed against 10 mM Tris, 0.5 % sucrose. Dialyzed sample was lyophilized and again solubilized in MQ (2 ml final volume). Solubilized sample was centrifuged at 12k rpm, 4 C, 10 min. Soup was subjected to gel filtration chromatography. 450 ml Sephacryl-100 was packed in Pharmacia K2.6/100 column to a height of 90 cm with a flow rate of 2 ml/min. Buffer used for chromatography was 50 mM Tris, pH 8.5, 200 mM NaCl. Single peak elute was collected. Eluted protein was dialyzed against 10 mM Phosphate, pH 7.5. Homogeneity and concentration of dialyzed elute was checked by SDS-PAGE, micro BCA (Pierce). Dialyzed sample was lyophilized and used for further characterization and bioactivity tests. Details of recovery of bioactive r-hGH after solubilization of n-propanol based buffer are provided in Table 3.

### Example 6

### Obtaining bioactive recombinant human growth hormone (r-hGH)

Recombinant human growth hormone (r-hGH) was solubilized in solubilization buffer comprising 6 M propanol, 2 M urea, 50 mM Tris buffer and was refolded by pulsatile dilution method (S. Grudzielanek, R. Jansen, R. Winter, Solvational tuning of the unfolding, aggregation and amyloidogenesis of insulin, J.Mol.Biol. 351, 2005, 879-894). The solubilized r-hGH (5 mg/ml) was added drop wise in discontinuous manner in to 90 ml of ice cold refolding buffer (pH 8.5) comprising 50 mM Tris, 5 mM EDTA, 2M urea, 10% sucrose and 1mM PMSF in such a way that the concentration of unfolded protein was always less than 50 µg/ml. Total 10 ml of the solubilized r-hGH (50 mg protein) was refolded in to 100 ml of the refolding buffer. The final pH of the refolded protein solution was adjusted to 8.5 using IN HCl.

Refolded r-hGH was loaded onto DEAE sepharose column and eluted with a gradient of 0.1- 0.5 M NaCl in the refolding buffer at a flow rate of 2 ml/min. The fractions which show a single r-hGH protein band on SDS-PAGE were pooled together, dialyzed against 10 mM Tris buffer at pH 8.5, 0. 25 % sucrose in order to remove urea and then lyophilized. The lyophilized r-hGH solubilized in 5 ml mill Q water and loaded onto a Sephacryl S-100 column (bed volume = 90 cm X1.6 cm, flow rate of 20 ml/hr) for further purification. Pure r-hGH fractions were pooled and lyophilized for physico-chemical and bioactivity assays and were stored at -20° C for further use.

### SDS-PAGE

The cell lysate, isolated inclusion bodies and purified r-hGH were resolved on a 12% polyacrylamide gel containing 0.1% SDS and stained with Coomassie brilliant blue stain as described previously. r-hGH was electrophoretically blotted on to a 0.45µm nitrocellulose membrane (Trans-Blot Transfer Medium, Amersham Pharmacia Laboratories, USA) and were probed with anti-rabbit monoclonal antibody generated against ovine growth hormone as described previously (R.J. St. John, J.F. Carpenter, T.W. Randolph, High pressure fosters protein refolding from aggregates at high concentrations, Proc.Natl.Acad.Sci.U.S.A 96 (1999) 13029-13033).

### Characterization of r-hGH by Spectroscopic Analysis

A CD spectrum of r-hGH in 10mM Tris buffer, pH 8.5 was obtained at 25 ° C in the wavelength range of 190-250 nm using a JASCO-710 Spectropolarimeter. The sample was scanned for data accumulation and the average values were plotted.

UV spectrum of r-hGH was scanned within the wavelength range 240-300nm (Kontron UV-Visible Spectrophotometer). A fluorescence emission spectrum was obtained by exciting protein molecules at 280 nm and measuring fluorescence in the wavelength range 300-350 nm using a spectrofluorometer (Shimadzu Model 1501).

### NB2 cell assays

NB2 lymphoma cell line was maintained in complete RPMI-1640 (10% HS, 10% FCS) media. Cell were arrested in G₀-G₁ phase by reducing FCS to a final concentration of 1%, 10% HS. 0.5X10⁵ cells per well were taken in 96 well plate to study the growth promoting action of purified r-hGH and commercially obtained human growth hormone from Boehringer Mannheim (Std). Cells were grown in different conditions (RPMI only, RPMI + 10ng/ml BSA, RPMI + 10ng/ml std hGH, RPMI + purified r-hGH) and cells were counted at different time points (0hr-120hrs.) Growth assay was carried out in duplicate with 5% CO₂, 37°C.

### Example 7

### Obtaining bioactive recombinant bovine growth hormone

Recombinant bovine growth hormone was solubilized in solubilization buffer comprising 6 M propanol, 2 M urea, 50 mM Tris buffer and was refolded by pulsatile dilution method (S. Grudzielanek, R. Jansen, R. Winter, Solvational tuning of the unfolding, aggregation and amyloidogenesis of insulin, J.Mol.Biol. 351, 2005, 879-894). The solubilized bovine growth hormone (5 mg/ml) was added drop wise in discontinuous manner in to 90 ml of ice cold refolding buffer (pH 8.5) comprising 50 mM Tris, 5 mM EDTA, 2M urea, 10% sucrose and 1mM PMSF in such a way that the concentration of unfolded protein was always less than 50 µg/ml. Total 10 ml of the solubilized bovine growth hormone (50 mg protein) was refolded in to 100 ml of the refolding buffer. The final pH of the refolded protein solution was adjusted to 8.5 using IN HCl.

Refolded bovine growth hormone was loaded onto DEAE sepharose column and eluted with a gradient of 0.1- 0.5 M NaCl in the refolding buffer at a flow rate of 2 ml/min. The fractions which show a single bovine growth hormone protein band on SDS-PAGE were pooled together, dialyzed against 10 mM Tris buffer at pH 8.5, 0. 25 % sucrose in order to remove urea and then lyophilized. The lyophilized bovine growth hormone solubilized in 5 ml mill Q water and loaded onto a Sephacryl S-100 column (bed volume = 90 cm X1.6 cm, flow rate of 20 ml/hr) for further purification. Pure bovine growth hormone fractions were pooled and lyophilized for physico-chemical and bioactivity assays and were stored at -20° C for further use.

### Example 8

### Obtaining bioactive recombinant human super oxide dismutase (SOD)

Human superoxide dismutase (SOD) was expressed as inclusion bodies in *E. coli.* The recombinant SOD was solubilized and refolded in to bioactive form using this method. Fed-batch fermentation of *E. coli* resulted in production of 2 g/l of recombinant SOD as inclusion bodies. The denatured recombinant SOD in form of inclusion bodies were isolated and purified from the cell by the lysis and centrifugation.

Recombinant human super oxide dismutase (SOD) was solubilized in solubilization buffer comprising 6 M propanol, 2 M urea, 50 mM Tris buffer and was refolded by pulsatile dilution method (S. Grudzielanek, R. Jansen, R. Winter, Solvational tuning of the unfolding, aggregation and amyloidogenesis of insulin, J.Mol.Biol. 351, 2005, 879-894). The solubilized recombinant SOD was separated by high speed centrifuge. Maximum solubility of recombinant SOD in propanol based buffer was 3 mg/ml. The solubilized human SOD (5 mg/ml) was added drop wise in discontinuous manner in to 90 ml of ice cold refolding buffer (pH 8.5) comprising 50 mM Tris, 5 mM EDTA, 2M urea, 10% sucrose and 1mM PMSF in such a way that the concentration of unfolded protein was always less than 50 µg/ml. Total 10 ml of the solubilized SOD (50 mg protein) was refolded into 100 ml of the refolding buffer. The final pH of the refolded protein solution was adjusted to 8.5 using IN HCl.

Refolded SOD was loaded onto DEAE sepharose column and eluted with a gradient of 0.1- 0.5 M NaCl in the refolding buffer at a flow rate of 2 ml/min. The fractions which show a single human SOD protein band on SDS-PAGE were pooled together, dialysed against 10 mM Tris buffer at pH 8.5, 0. 25 % sucrose in order to remove urea and then lyophilized. The lyophilized SOD solubilized in 5 ml mill Q water and loaded onto a Sephacryl S-100 column (bed volume = 90 cm X1.6 cm, flow rate of 20 ml/hr) for further purification. Pure human SOD fractions were pooled and lyophilized for physico-chemical and bioactivity assays and were stored at -20° C for further use. The propanol based solubilization of inclusion body protein results in high recovery of bioactive SOD.

### Example 9

### Obtaining bioactive Mycobacterium tuberculosis derived recombinant enolase

Recombinant *enolase* was solubilized in solubilization buffer comprising 6 M propanol, 2 M urea, 50 mM Tris buffer and was refolded by pulsatile dilution method (S. Grudzielanek, R. Jansen, R. Winter, Solvational tuning of the unfolding, aggregation and amyloidogenesis of insulin, J.Mol.Biol. 351, 2005, 879-894). The solubilized *enolase* (5 mg/ml) were added drop wise in discontinuous manner in to 90 ml of ice cold refolding buffer (pH 8.5) comprising 50 mM Tris, 5 mM EDTA, 2M urea, 10% sucrose and 1 mM PMSF in such a way that the concentration of unfolded protein was always less than 50 µg/ml. Total 10 ml of the solubilized *enolase* (50 mg protein) was refolded in to 100 ml of the refolding buffer. The final pH of the refolded protein solution was adjusted to 8.5 using IN HCl.

Refolded *enolase* was loaded onto DEAE sepharose column and eluted with a gradient of 0.1- 0.5 M NaCl in the refolding buffer at a flow rate of 2 ml/min. The fractions which show a single *enolase* protein band on SDS-PAGE were pooled together, dialyzed against 10 mM Tris buffer at pH 8.5, 0. 25 % sucrose in order to remove urea and then lyophilized. The lyophilized r-hGH solubilized in 5 ml mill Q water and loaded onto a Sephacryl S-100 column (bed volume = 90 cm X1.6 cm, flow rate of 20 ml/hr) for further purification. Pure *enolase* fractions were pooled and lyophilized for physico-chemical and bioactivity assays and were stored at -20° C for further use.

### Example 10

### Obtaining bioactive Mycobacterium tuberculosis derived recombinant pyruvate kinase

Recombinant *pyruvate kinase* was solubilized in solubilization buffer comprising 6 M propanol, 2 M urea, 50 mM Tris buffer and was refolded by pulsatile dilution method (S. Grudzielanek, R. Jansen, R. Winter, Solvational tuning of the unfolding, aggregation and amyloidogenesis of insulin, J.Mol.Biol. 351, 2005, 879-894). The solubilized *enolase* and *pyruvate kinase* (5 mg/ml) were added drop wise in discontinuous manner in to 90 ml of ice cold refolding buffer (pH 8.5) comprising 50 mM Tris, 5 mM EDTA, 2M urea, 10% sucrose and 1mM PMSF in such a way that the concentration of unfolded protein was always less than 50 µg/ml. Total 10 ml of the solubilized *pyruvate kinase* (50 mg protein) was refolded in to 100 ml of the refolding buffer. The final pH of the refolded protein solution was adjusted to 8.5 using IN HCl. Refolded *pyruvate kinase* was loaded onto DEAE sepharose column and eluted with a gradient of 0.1- 0.5 M NaCl in the refolding buffer at a flow rate of 2 ml/min. The fractions which show a single *pyruvate kinase* protein band on SDS-PAGE were pooled together, dialyzed against 10 mM Tris buffer at pH 8.5, 0. 25 % sucrose in order to remove urea and then lyophilized. The lyophilized r-hGH solubilized in 5 ml mill Q water and loaded onto a Sephacryl S-100 column (bed volume = 90 cm X1.6 cm, flow rate of 20 ml/hr) for further purification. Pure *pyruvate kinase* fractions were pooled and lyophilized for physico-chemical and bioactivity assays and were stored at -20° C for further use.

**Table 1: Composition of buffer having different alkyl alcohols and relative absorbance at 280 nm after solubilization of IBs of r-hGH in corresponding buffer**

| **S. No** | **Solubilization buffer** | **OD at 280** |
|---|---|---|
| 1 | Tris 50 mM, pH 8.5, Methanol 6 M and Urea 2 M | 0.01 |
| 2 | Tris 50 mM, pH 8.5, Ethanol 6 M and Urea 2 M | 0.01 |
| 3 | Tris 50 mM, pH 8.5, n-Propanol 6 M and Urea 2 M | 1.42 |
| 4 | Tris 50 mM, pH 8.5, Ethylene glycol 6 M and Urea 2 M | 0.01 |
| 5 | Tris 50 mM, pH 8.5, Glycerol 6 M and Urea 2 M | 0.01 |
| 6 | Tris Tris 50 mM, pH 8.5, n-Butanol 6 M and Urea 2 M | Immiscible |

**Table 2: Comparative refolding yield of r-hGH after solubilization in different solubilization buffers**

| **S. No** | **Solubilization buffers** | **O.D. at 280 nm after solubilization** | **Refolding buffers** | **O.D at 280 nm after refolding** | **Refolding (%)** |
|---|---|---|---|---|---|
| 1 | Tris 50 mM, pH 8.5, GdmCl 6 M | 0.422 | Tris 100 mM, pH 8.5, GdmCl 1 M, Sucrose 5% | 0.96 | 22.7 |
| 2 | Tris 50 mM, pH 8.5, Urea 8 M | 0.412 | Tris 100 mM, pH 8.5, Urea 2 M, Sucrose 5 % | 0.205 | 49.7 |
| 3 | Tris 50 mM, pH 12, Urea 2 M | 0.432 | Tris 100 mM, pH 8.5, Urea 2 M, Sucrose 5 % | 0.239 | 55.3 |
| 4 | Tris 50 mM, pH 8.5, n-Propanol 6 M, Urea 2 M | 0.334 | Tris 100 mM, pH 8.5, Urea 2 M, Sucrose 5 % | 0.257 | 76.9 |

**Table 3: Recovery of bioactive r-hGH after solubilization of n-propanol based buffers**

| **Steps** | **Total protein (mg)** | **Step yield** | **Overall yield** |
|---|---|---|---|
| **Solubilization** | 47 mg | | |
| **Refolding** | 34 mg | 72% | 72.3 % |
| **Ion exchange chromatography** | 26 mg | 76% | 55 % |
| **Gel filtration chromatography** | 21.1 mg | 81% | 44.8 % |

## Claims

1. A process for obtaining a bioactive recombinant protein from inclusion bodies, said process comprising:
a. solubilizing inclusion bodies in a solubilization buffer comprising Tris, urea, and propanol to obtain a suspension;
b. incubating said suspension at room temperature; and
c. adding refolding buffer comprising Tris, urea and sucrose to said suspension to obtain the bioactive recombinant protein,
wherein said solubilization buffer comprises 2 M urea and 4-8 M propanol.

2. The process as claimed in claim 1, wherein said recombinant protein is expressed in a host cell selected from a group consisting of *E. coli,* yeast and a eukaryotic cell.

3. The process as claimed in claim 2, wherein said eukaryotic cell is selected from a group consisting of an insect cell, an animal cell and a plant cell.

4. The process as claimed in claim 1, wherein said recombinant protein is selected from a group consisting of growth factors, interferons, interleukins, hormones, antibody fragments, human growth hormone, bovine growth hormone, human *super oxide dismutase* (SOD) and *Mycobacterium tuberculosis* derived recombinant *enolase* and *pyruvate kinase.*

5. The process claimed in claim 1, wherein said solubilization buffer comprises 50-100 mM Tris, 2 M urea, 6 M n-Propanol, 5-10% sucrose, 0.5 mM EDTA and 1 mM PMSF.

6. The process as claimed in claim 1, wherein said solubilization buffer comprises 50-100 mM Tris, 2 M urea and 6 M n-Propanol.

7. The process as claimed in claim 1, wherein the pH of the solubilization buffer is 8.5.

8. The process as claimed in claim 1, wherein said incubation is carried out for 30 minutes.

9. The process as claimed in claim 1, wherein the refolding buffer comprises 50-100 mM Tris, 5 mM EDTA, 2 M urea, 5-10% sucrose and 1 mM PMSF.

10. The process as claimed in claim 1, wherein the pH of the refolding buffer is 8.5.

## Patentansprüche

1. Verfahren zur Gewinnung eines bioaktiven rekombinanten Proteins aus Einschlusskörpern, wobei das Verfahren umfasst:
a. Solubilisieren der Einschlusskörper in einem Solubilisierungspuffer umfassend Tris, Harnstoff und Propanol, um eine Suspension zu erhalten;
b. Inkubieren der Suspension bei Raumtemperatur; und
c. Hinzufügen eines Rückfaltungspuffers umfassend Tris, Harnstoff und Sucrose zu der Suspension, um das bioaktive rekombinante Protein zu erhalten,
wobei der Solubilisierungspuffer 2 M Harnstoff und 4-8 M Propanol umfasst.

2. Verfahren wie in Anspruch 1 beansprucht, wobei das rekombinante Protein in einer Wirtszelle exprimiert wird, die ausgewählt ist aus der Gruppe bestehend aus *E. coli,* Hefe und einer eukaryotischen Zelle.

3. Verfahren wie in Anspruch 2 beansprucht, wobei die eukaryotische Zelle ausgewählt ist aus einer Gruppe bestehend aus einer Insektenzelle, einer Tierzelle und einer Pflanzenzelle.

4. Verfahren wie in Anspruch 1 beansprucht, wobei das rekombinante Protein ausgewählt ist aus einer Gruppe bestehend aus Wachstumsfaktoren, Interferonen, Interleukinen, Hormonen, Antikörperfragmenten, humanen Wachstumshormon, Rinder-Wachstumshormon, humaner *Superoxiddismutase* (SOD) und aus *Mykobakterium tuberkulosis* gewonnener rekombinanter *Enolase* und *Pyruvatkinase.*

5. Verfahren wie in Anspruch 1 beansprucht, wobei der Solubilisierungspuffer 50-100 mM Tris, 2 M Harnstoff, 6 M n-Propanol, 5-10% Sucrose, 0,5 mM EDTA und 1 mM PMSF umfasst.

6. Verfahren wie in Anspruch 1 beansprucht, wobei der Solubilisierungspuffer 50-100 mM Tris, 2 M Harnstoff und 6 M n-Propanol umfasst.

7. Verfahren wie in Anspruch 1 beansprucht, wobei der pH des Solubilisierungspuffer 8,5 ist.

8. Verfahren wie in Anspruch 1 beansprucht, wobei die Inkubation für 30 Minuten ausgeführt wird.

9. Verfahren wie in Anspruch 1 beansprucht, wobei der Rückfaltungspuffer 50-100 mM Tris, 5 mM EDTA, 2 M Harnstoff, 5-10% Sucrose und 1 mM PMSF umfasst.

10. Verfahren wie in Anspruch 1 beansprucht, wobei der pH des Rückfaltungspuffers 8,5 ist.

## Revendications

1. Procédé d'obtention d'une protéine recombinante bioactive à partir de corps d'inclusion, ledit procédé comprenant :
a. la solubilisation de corps d'inclusion dans un tampon de solubilisation contenant du Tris, de l'urée, et du propanol pour obtenir une suspension ;
b. l'incubation de ladite suspension à température ambiante ; et
c. l'ajout d'un tampon de repliement contenant du Tris, de l'urée et du sucrose à ladite suspension pour obtenir la protéine recombinante bioactive,
où ledit tampon de solubilisation contient 2 M d'urée et 4-8 M de propanol.

2. Le procédé tel que revendiqué dans la revendication 1, où ladite protéine recombinante est exprimée dans une cellule hôte sélectionnée dans un groupe constitué par E. *coli,* de la levure et une cellule eucaryote.

3. Le procédé tel que revendiqué dans la revendication 2, où ladite cellule eucaryote est sélectionnée dans un groupe constitué par une cellule d'insecte, une cellule animale et une cellule végétale.

4. Le procédé tel que revendiqué dans la revendication 1, où ladite protéine recombinante est sélectionnée dans un groupe constitué par des facteurs de croissance, des interférons, des interleukines, des hormones, des fragments d'anticorps, une hormone de croissance humaine, une hormone de croissance bovine, la superoxyde dismutase (SOD) humaine et l'énolase et la pyruvate kinase recombinantes dérivées de *Mycobacterium tuberculosis.*

5. Le procédé revendiqué dans la revendication 1, où ledit tampon de solubilisation contient 50-100 mM de Tris, 2 M d'urée, 6 M de n-propanol, 5-10 % de sucrose, 0,5 mM d'EDTA et 1mM de PMSF.

6. Le procédé tel que revendiqué dans la revendication 1, où ledit tampon de solubilisation contient 50-100 mM de Tris, 2 M d'urée, 6 M de n-propanol.

7. Le procédé tel que revendiqué dans la revendication 1, où le pH du tampon de solubilisation est de 8,5.

8. Le procédé tel que revendiqué dans la revendication 1, où ladite incubation est effectuée pendant 30 minutes.

9. Le procédé tel que revendiqué dans la revendication 1, où ledit tampon de repliement contient 50-100 mM de Tris, 5 mM d'EDTA, 2 M d'urée, 5-10 % de sucrose et 1mM de PMSF.

10. Le procédé tel que revendiqué dans la revendication 1, où le pH du tampon de repliement est de 8,5.
